# EUROPEAN PATENT APPLICATION

(11) **EP 1 935 327 A1**
(43) Date of publication of application: **25.06.2008**
(21) Application number: 07024560.0
(22) Date of filing: 18.12.2007
(51) Int. Cl.: A61B 1/05

(54) **Endoscope apparatus**

(30) Priority: 19.12.2006 JP 2006341821
(71) Applicant: Olympus Corporation, Shibuya-ku Tokyo (JP)
(72) Inventor: Yoshizumi, Naoyuki, Kanagawa 229-0001 (JP); Kotouda, Kaoru, Tokyo 151-0072 (JP)
(74) Representative: Winter, Brandl, Fürniss, Hübner Röss, Kaiser, Polte Partnerschaft Patent- und Rechtsanwaltskanzlei

(57) **Abstract**

An endoscope apparatus to which an endoscope is detachably connected has an endoscope interface, an image processor and a parameter transfer control section. Here, the endoscope multiplexes a video signal obtained by an image pickup section with an endoscope ID as endoscope identification information, and transmits the signal to the endoscope apparatus. The endoscope interface separates the endoscope ID from the signal from the endoscope. The image processor has a parameter register in which a parameter related to an image process of the video signal is set, and a functional module for performing a predetermined image process with respect to the video signal based on the parameter. The parameter transfer control section reads the parameter corresponding to the endoscope ID separated in the endoscope interface, from a table memory for storing the parameter for each endoscope ID, and transfers the parameter to the parameter register.

## Description

### BACKGROUND OF THE INVENTION

### 1. Field of the Invention

The present invention relates to an endoscope apparatus which can accommodate multiple types of endoscopes.

### 2. Description of the Related Art

In endoscope apparatuses, there are dedicated endoscope apparatuses depending on purposes such as for surgery and for digestive apparatuses, and dedicated electronic endoscopes (abbreviated as "scopes") have been used in those endoscope apparatuses.

In recent years, there has been developed an endoscope apparatus in which the dedicated scope for the surgery, for the digestive apparatuses or the like is connected to one general purpose endoscope apparatus and used as a scope depending on an operation.

Since image sizes, color characteristics and the like vary for each scope type, a video data processing section (hereinafter referred to as "processor") in such an endoscope apparatus has performed setting depending on a used scope by setting switches and the like to select parameters embedded in hardware whenever a power is turned on or the scope is changed, and thereby an image process optimal for the connected scope has been performed.

In this case, as means of reducing errors in the setting and the like by users, Japanese Patent No. 2693978 discloses a technique for automatically generating a threshold level of an AGC gain from a unique ID showing a scope kind (scope ID).

Similarly, Japanese Patent No. 2002-200039 discloses a technique for selecting a CCD driving signal generation circuit for each scope which has been previously embedded in a circuit within the processor, based on the scope ID.

In the general purpose endoscope apparatus, in the case of a few types of scopes, there is no problem in automatically generating a threshold of the AGC gain or selecting the CCD driving signal generation circuit based on the scope ID of the connected scope.

However, in order to accommodate many types of scopes depending on application in the operation, it is necessary to embed all generation circuits depending on respective characteristics, in the hardware. Furthermore, it means that options (circuits) and selectors depending on many types of scopes are included for many image processes in the processor. As a result, a circuit size increases.

Furthermore, if the characteristics of the scope are improved, a new scope is added or the like after the endoscope apparatus has been marketed, replacement or improvement of the endoscope apparatus imposes a burden on the users or makers.

As measures against these problems, it is conceivable to have means of causing a scope side to have all necessary information and transferring parameter information from the scope to the processor.

However, in the scope, since a diameter of the scope is required to be as small as possible for the purpose of reducing a burden on patients, the number of transmission lines from a CCD is required to be one to several, and thereby it is difficult to provide an information transmission path separately from a video signal transmission path.

Consequently, for example, means of time-division multiplexing a video signal with the information and serially transmitting them is employed for information transmission from the scope to the processor. In the method, since the information is multiplexed in an idle time for the video signal, a device for dividing a large amount of information into several frames and transmitting the frames or the like is required under temporal constraints.

### SUMMARY OF THE INVENTION

The present invention has been made in view of the above described circumstances, and it is an object of the present invention to provide an endoscope apparatus which can accommodate types of endoscopes (scopes).

Furthermore, it is another object of the present invention to provide an endoscope apparatus which suppresses an increase in a circuit size and has extensibility.

An endoscope apparatus according to the present invention to which an endoscope for transmitting a signal in which a video signal obtained by an image pickup section has been multiplexed with an endoscope ID as an endoscope identification signal is detachably connected includes an endoscope interface for separating the endoscope ID from the transmitted signal which has been received, an image processor having a parameter register in which a parameter related to an image process of the video signal is set and at least one functional module for performing a predetermined image process with respect to the video signal based on the parameter set in the parameter register, a table memory for storing the parameter suitable for the endoscope corresponding to the endoscope ID for each endoscope ID, and a parameter transfer control section for reading the parameter corresponding to the endoscope ID separated in the endoscope interface, from the table memory, and transferring the parameter to the parameter register.

### BRIEF DESCRIPTION OF THE DRAWINGS

Fig. 1 is a configuration diagram showing an entire configuration of an endoscope apparatus according to a first embodiment of the present invention;
Fig. 2a is diagram showing an example of a CCD outputted signal in which a scope ID and unit information have been multiplexed;
Fig. 3 is a diagram showing an example of the CCD outputted signal of a multiplexing example different from Fig. 2;
Fig. 4A is a block diagram showing one configuration example of a processor;
Fig. 4B is a block diagram showing another configuration example of the processor;
Fig. 5 is a block diagram showing a configuration of a periphery of a system bus including an image processor within the processor;
Fig. 6 is a flowchart showing an example of an operating procedure according to the first embodiment;
Fig. 7 is a block diagram showing a configuration of the periphery of the system bus including the image processor within the processor of a variation of the first embodiment;
Fig. 8 is an explanatory diagram showing a space allocation example in a table ROM;
Fig. 9 is a configuration diagram showing an entire configuration of the endoscope apparatus according to a second embodiment of the present invention;
Fig. 10 is a block diagram showing a configuration of the image processor;
Fig. 11 is a flowchart showing an example of an operating procedure according to the second embodiment;
Fig. 12 is a configuration diagram showing an entire configuration of the endoscope apparatus having the table ROM configured to be on-board rewritable;
Fig. 13 is a block diagram showing a configuration of the periphery of the system bus in the processor according to a third embodiment of the present invention; and
Fig. 14 is a block diagram showing a configuration of the periphery of the system bus in the processor according to a variation of the third embodiment of the present invention.

### DETAILED DESCRIPTION OF THE PREFERRED EMBODIMENTS

Embodiments of the present invention will be described below with reference to the drawings.

### (First Embodiment)

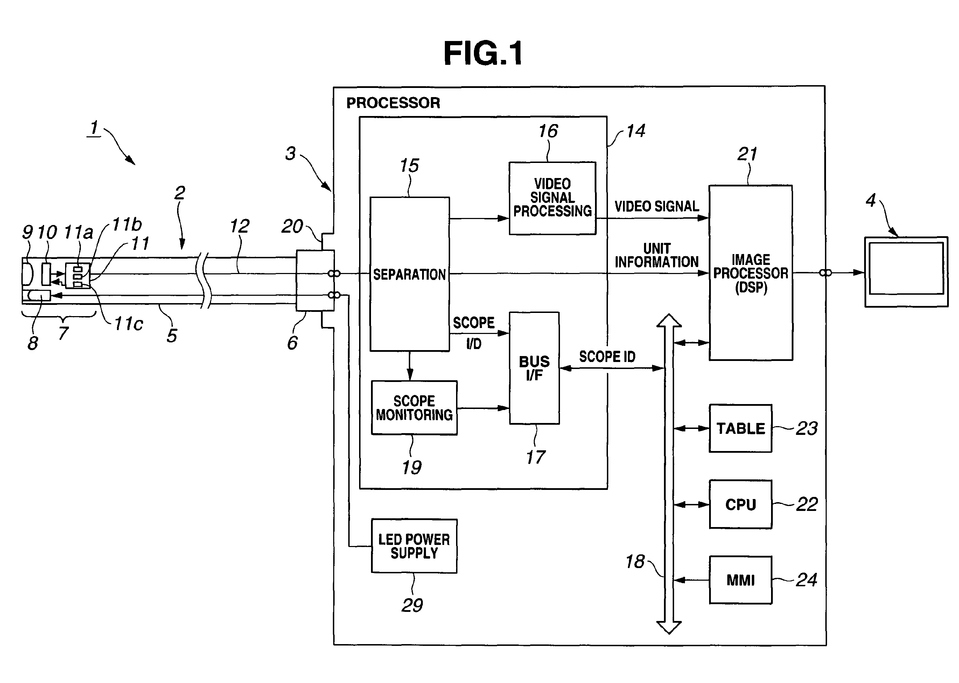

Fig. 1 shows an entire configuration of an endoscope apparatus of a first embodiment of the present invention. The endoscope apparatus 1 includes an electronic endoscope (hereinafter simply abbreviated as "scope") 2 inserted into a body cavity, a processor 3 as a signal processing apparatus to which the scope 2 is detachably connected and which includes a video data processing section (image processing section) and the like, and a monitor 4 for displaying an endoscope image corresponding to a video signal outputted from the processor 3.

The scope 2 has an elongated insertion portion 5, and at a rear end of the insertion portion 5, a scope connector 6 which is detachably connected to a connector acceptor 20 of the processor 3 is provided. It should be noted that a bendable bending portion (not shown) is provided at a rear end of a distal end portion 7 of the insertion portion 5.

At the distal end portion 7 of the above described insertion portion 5, an illumination window and an observation window are provided. Inside the illumination window, for example, a white color LED (Light-Emitting Diode) 8 is provided. The white color LED 8 is turned on by driving electricity supplied via a driving line and emits a white color illumination light from the illumination window via an illumination lens.

The illumination light emitted from the illumination window illuminates an inside of the body cavity inserted with the insertion portion 5. An optical image of the illuminated inside of the body cavity is imaged, for example, on a charge-coupled device (abbreviated as "CCD") 10 as a solid image pickup device placed at its imaging position, via an objective lens 9 attached to the observation window.

Moreover, within the distal end portion 7, a multiplexing section 11 for performing multiplexing with respect to an outputted signal of the CCD 10 is provided. In the CCD 10, for example, a CCD driving signal supplied from a CCD driving circuit 11c within the multiplexing section 11 is applied, and a photoelectrically converted signal charge is read and outputted as an image pickup signal (CCD outputted signal). It should be noted that the CCD driving circuit 11c may be configured to be provided at the processor 3 side.

The image pickup signal is inputted to a multiplexing circuit 11a within the multiplexing section 11. The multiplexing circuit 11a multiplexes the inputted image pickup signal with a scope ID and scope unit information (or device information, hereinafter simply abbreviated as "unit information") outputted from a ROM 11b provided within the multiplexing section 11.

It should be noted that the scope ID represents identification information for identifying each scope 2, and the identification information also includes information on a type of the scope 2.

Moreover, the unit information is information including the number of pixels of the CCD 10 as a device mounted on each scope 2, characteristics such as spectral sensitivity of the CCD 10, characteristics of the objective lens 9 and the like.

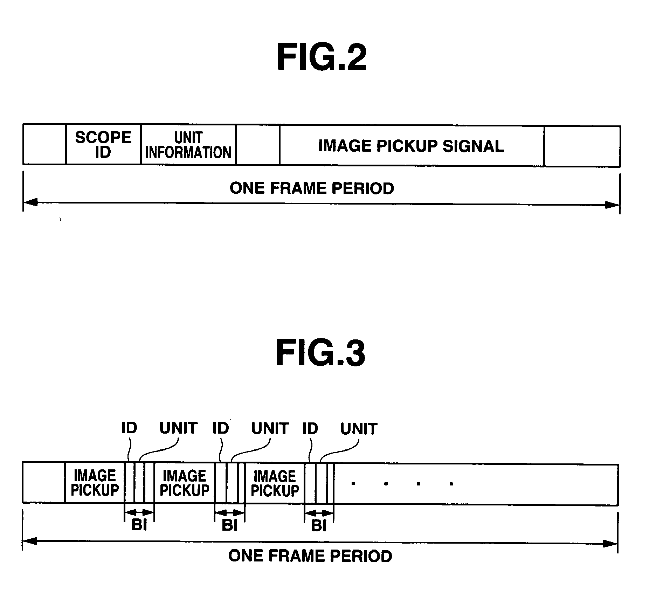

The multiplexed signal which has been multiplexed is transmitted to the processor 3 via a transmission line 12 inserted through the insertion portion 5. Fig. 2 shows a signal example of one frame of the multiplexed signal transmitted to the processor 3 via the transmission line 12.

As shown in Fig. 2, the scope ID, the unit information and the image pickup signal (CCD outputted signal) have been time-division multiplexed to form the multiplexed signal.

Fig. 2 shows, for example, an example in which the scope ID, the unit information and the image pickup signal have been multiplexed separately in three periods within one frame period. However, the multiplexing may be performed as shown in Fig. 3.

In Fig. 3, for example, multiple blanking periods BI have been provided for the image pickup signal in one frame period, and the multiplexing has been performed so that the scope ID and the unit information are arranged in the respective blanking periods BI. It should be noted that, as will be described below, instead of the image pickup signal, the video signal in which a signal process (image process) has been performed with respect to the image pickup signal may be multiplexed with the scope ID and the unit information.

As shown in Fig. 1, the multiplexed signal is inputted to a scope interface (abbreviated as "scope I/F") 14 provided in the processor 3, and the multiplexed signal is separated into the image pickup signal, the scope ID and the unit information by a separation block 15 provided in the scope I/F 14.

Moreover, the image pickup signal separated by the separation block 15 is inputted to a video signal processing block 16, applied with a process such as filtering for eliminating unnecessary components, and further A/D converted to generate a digital video signal. The video signal is outputted to an image processor 21 for performing the image process or a video data process (depending on parameters), along with the unit information.

It should be noted that if the video signal has been multiplexed with the scope ID and the unit information as described above, the multiplexed signal will be separated into the video signal, the scope ID and the unit information by the scope I/F 14.

Moreover, the scope ID is outputted to a control CPU (hereinafter simply abbreviated as "CPU (Central Processing Unit)") 22 which is connected to a system bus 18 and has a function of control means, via a bus I/F 17.

The CPU 22 performs control of the respective sections within the processor 3, for example, such as parameter transfer control for transferring a corresponding parameter from a table ROM 23 described below into the image processor 21 depending on the scope ID.

To the system bus 18, the table ROM (hereinafter simply abbreviated as "table") 23 for storing the parameters required for the image process in the image processor 21, and a man-machine interface (abbreviated as "MMI" in the drawings) 24 such as a keyboard or the like serving as a user interface are connected.

A user can specify or select an operating mode when the image process is performed by the image processor 21, via the man-machine interface 24.

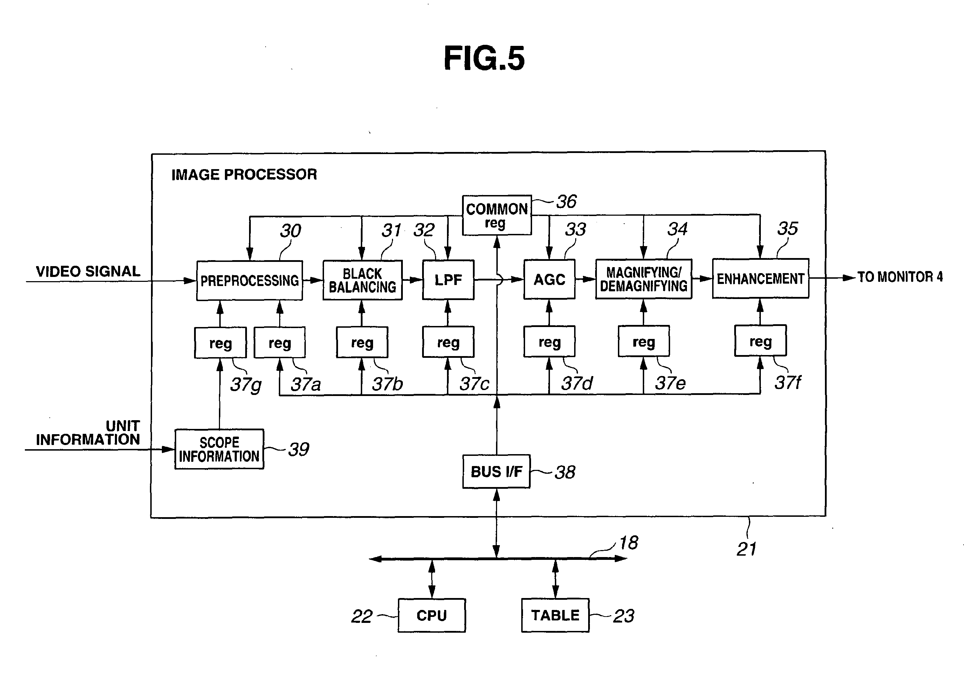

The image processor 21 can be configured with, for example, a digital signal processor (DSP) and the like, and includes multiple functional modules for performing various image processes as shown in Fig. 5 which will be described below.

The respective functional modules are connected to dedicated registers respectively, and perform the image processes depending on the parameters set (written) in the register (for example, with hardware or software not shown).

It should be noted that, as described below, the table 23 in the present embodiment is configured with a semiconductor memory which is nonvolatile and can electrically rewrite data, such as an EEPROM or a flash memory.

Moreover, a scope monitoring block 19 is connected to the separation block 15, and the scope monitoring block 19 monitors whether or not the scope connector 6 is connected to the connector acceptor 20. Then, the scope monitoring block 19 outputs a result of the monitoring to the CPU 22 via the bus I/F 17.

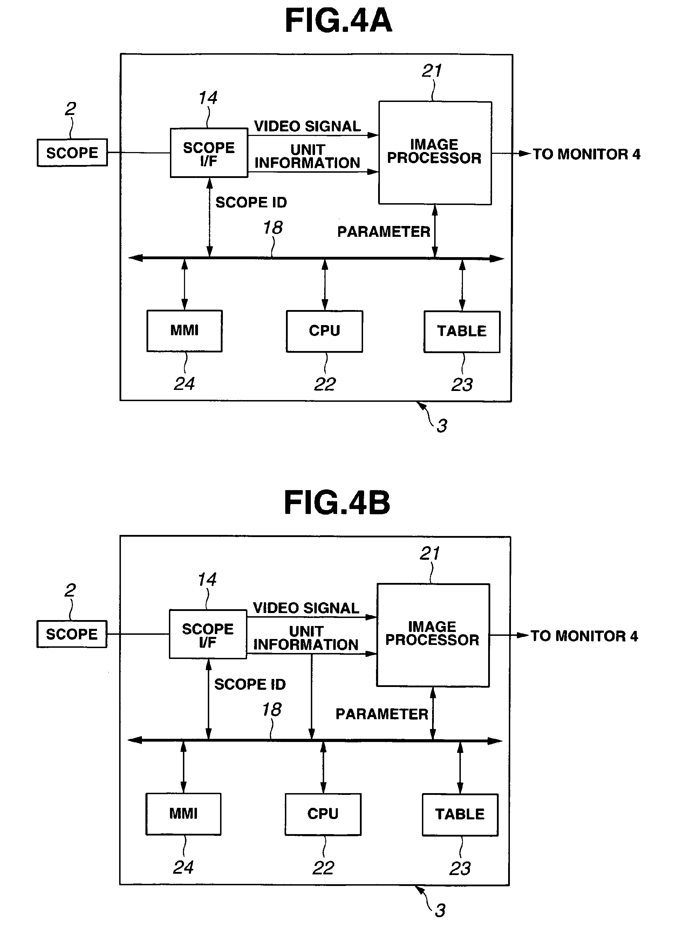

Fig. 4A shows one configuration example of the processor 3. It should be noted that the scope I/F 14 in Fig. 1 is shown as one block in Fig. 4A. Moreover, an LED driving circuit 29 for driving the LED 8 shown in Fig. 1 is omitted.

The video signal and the unit information which are separated within the scope I/F 14 and outputted from the scope I/F 14 are inputted to the image processor 21 for performing the image process depending on the unit information and the like. The video signal generated by the image processor 21 is outputted to the monitor 4. Then, the monitor 4 displays the endoscope image corresponding to the video signal.

Moreover, as described above, the scope ID which is separated within the scope I/F 14 and outputted from the scope I/F 14 is inputted to the CPU 22.

It should be noted that, although Fig. 4A shows the configuration example in which only the scope ID is inputted to the CPU 22, the configuration may be modified so that the unit information is also inputted to the CPU 22 in addition to the scope ID as shown in Fig. 4B.

When a power is turned on, the CPU 22 recognizes (identifies) a type and the like of the scope 2 connected to the processor 3 by capturing the scope ID. Moreover, the CPU 22 recognizes a type, characteristics and the like of the CCD 10 mounted on the scope 2 by capturing the unit information.

Then, the CPU 22 reads the parameters corresponding to the scope 2 of a result of the recognition from the table 23, and outputs the parameters to the image processor 21. Then, the CPU 22 controls various processes in the image processor 21 as shown in Fig. 5 which will be described below so that processes suitable for the CCD 10 and the like mounted on the scope 2 which is actually connected thereto can be performed.

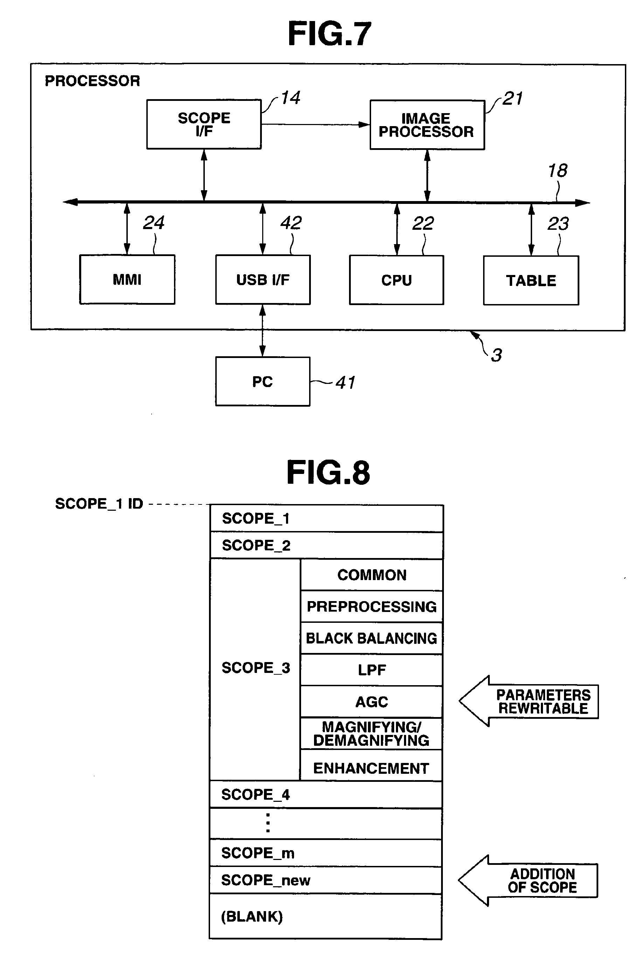

It should be noted that, in the table 23, depending on the type of the scope 2 as shown in Fig. 8 which will be described below, parameter data required in the case of performing the various image processes suitable for the type, the characteristics and the like of the CCD 10 mounted on the scope 2 has been previously stored. Moreover, addition, rewriting and the like of the parameter data can also be accommodated.

Fig. 5 shows a periphery of the image processor 21 and the system bus 18 within the processor 3 in Fig. 1.

As shown in Fig. 5, the image processor 21 includes the functional modules for performing the various image processes. The video signal is sequentially processed by a preprocessing section 30, a black balancing section 31, an LPF section 32, an AGC section 33, a magnifying/demagnifying section 34 and an enhancement section 35, and subsequently outputted to the monitor 4.

Moreover, the respective functional modules including the preprocessing section 30, the black balancing section 31, the LPF section 32, the AGC section 33, the magnifying/demagnifying section 34 and the enhancement section 35 are connected to a common register (abbreviated as "common reg" in Fig. 5) 36 and respective individual (dedicated) registers (abbreviated as "reg" in Fig. 5) 37a to 37f.

The common register 36 and the registers 37a to 37f are connected to the system bus 18 via the bus I/F 38.

Based on the scope ID at the time of turning on the power, the CPU 22 reads the corresponding parameters from the table 23, writes suitable processing parameters to the common register 36 and the registers 37a to 37f, and sets a state in which the processing parameters can be referred to by the respective functional modules such as the preprocessing section 30 to perform the processes.

It should be noted that only the preprocessing section 30 is further connected to an individual register 37g. In the register 37g, parameters read out from a scope information section 39 are set based on the unit information.

Thus, the respective functional modules including the preprocessing section 30, the black balancing section 31, the LPF section 32, the AGC section 33, the magnifying/demagnifying section 34 and the enhancement section 35 can suitably perform the various processes based on the parameters set in the common register 36 and the individual registers 37a to 37g, respectively.

Specifically, the preprocessing section 30 performs a preprocess such as color separation with respect to the inputted video signal, and outputs the signal to the black balancing section 31. The black balancing section 31 processes a black level balance for correcting a black level by using a signal of an optical black portion.

Moreover, when the signal outputted from the black balancing section 31 is inputted to the LPF 32, the LPF 32 applies a low pass filtering process to the signal and outputs the signal to the AGC section 33. The AGC section 33 performs auto gain control with respect to the inputted signal so that a gain is automatically adjusted depending on its level to maintain a suitable level.

Moreover, when the signal outputted from the AGC section 33 is inputted to the magnifying/demagnifying section 34, the magnifying/demagnifying section 34 performs a magnifying or demagnifying process with respect to the signal and outputs the signal to the enhancement section 35. The enhancement section 35 performs a process of edge emphasis in horizontal and vertical directions.

It should be noted that the user can specify the operating mode at the time of the image process such as a magnifying or demagnifying power (or its size) or an amount of the edge emphasis, via the man-machine interface 24. Then, for example, if the operating mode for the magnifying has been specified, the magnifying/demagnifying section 34 performs the magnifying process in a state with the suitable processing parameters corresponding to the CCD 10 which is actually connected and used.

Moreover, the scope monitoring block 19 monitors whether or not the scope connector 6 is connected to the connector acceptor 20, for example, based on the signal level and the like. Then, the scope monitoring block 19 outputs the monitoring result to the CPU 22 via the bus I/F 17.

When the power (of the processor 3) is turned on and the CPU 22 is activated, the CPU 22 detects whether or not the scope 2 is connected to the processor 3, based on the monitoring result at the scope monitoring block 19. If the scope 2 is connected to the processor 3, the CPU 22 sets the parameters corresponding to the scope 2 which is actually connected, in the image processor 21, based on the scope ID.

In other words, the CPU 22 has a function of a parameter transfer control section for reading the parameters corresponding to the scope 2 connected to the connector acceptor 20 of the processor 3, from the table 23 by using the scope ID, and transferring the read parameters to the registers 37a to 37f of the image processor 21.

Moreover, in an active state of the processor 3, the CPU 22 detects replacement of the scope 2 based on the monitoring result at the scope monitoring block 19. If the scope 2 has been replaced, the CPU 22 captures its scope ID again and sets the various parameters corresponding to the scope 2 in the registers within the image processor 21.

Thereby, even if the scope 2 has been replaced and connected, the configuration enables to perform suitable image processes corresponding to the replaced scope.

Moreover, if the user has specified or selected the operating mode, the CPU 22 controls to read the parameters corresponding to the operating mode from the table 23 and set (transfer) the parameters in the corresponding register. In other words, the CPU 22 has a function of an operating mode setting section (described below mainly in a third embodiment).

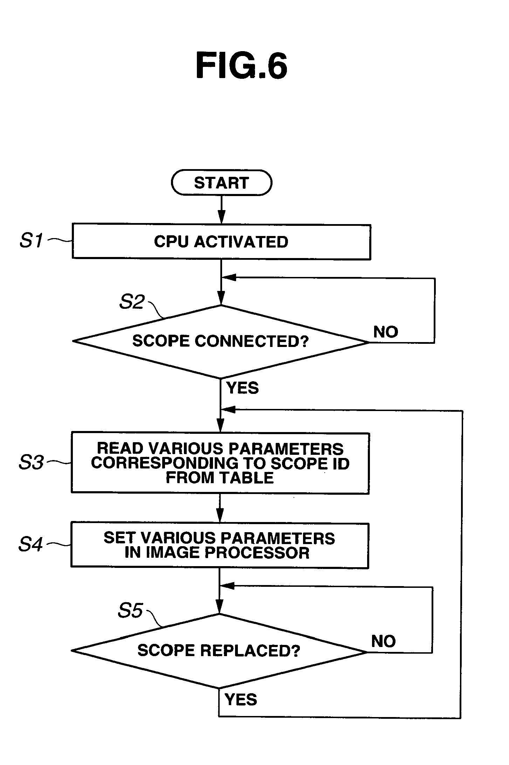

Fig. 6 is a flowchart showing schematic contents of operations of the processor 3 according to the present embodiment.

When the power of the processor 3 is turned on, the CPU 22 in the processor 3 is activated as shown in step S1.

Then, the CPU 22 detects whether or not the scope 2 is connected to the processor 3, based on the monitoring result at the scope monitoring block 19 as shown in step S2. Next, the CPU 22 comes to be in a state of waiting for the scope 2 to be connected.

When the scope 2 is connected, the CPU 22 captures the scope ID and reads the parameters corresponding to the scope 2 which is actually connected, from the table 23 as shown in step S3.

Then, as shown in step S4, the CPU 22 sets the read parameters in the common register 36 and the individual registers 37a to 37f within the image processor 21. Moreover, based on the scope ID, the corresponding parameters are set in the register 37g.

Thus, the image processor 21 comes to be in a state of performing the processes which are suitably corresponding to the CCD 10 mounted on the connected scope 2.

Moreover, also after step S4, the scope monitoring block 19 monitors the connection of the scope 2. The monitoring is continuously performed, for example, with a constant period. The scope monitoring block 19 outputs the monitoring result to the CPU 22.

Then, the CPU 22 determines whether or not the scope 2 has been replaced, based on the monitoring result as shown in step S5. If the scope 2 has not been replaced, the CPU 22 periodically performs the process at step S5, and if the scope 2 has been replaced, the CPU 22 returns to the process at step S3.

Then, the CPU 22 captures the scope ID again as described above, and sets the various parameters corresponding to the scope 2 in the image processor 21.

Thereby, even if the scope 2 has been replaced, the suitable image processes corresponding to the replaced scope 2 can be performed. Then, on a display surface of the monitor 4, the image picked up by the CCD 10 is displayed as the endoscope image. The user such as an operator can observe the endoscope image and smoothly perform an endoscopic diagnosis or an endoscopy.

Moreover, also if the user has instructed to change the operating mode, the CPU 22 sets the parameters to be changed so that the image processes are performed in that operating mode. Thereby, it is possible to accommodate the change of the operating mode, for example, such a case where the magnifying power has been changed in the operating mode for the image process of the magnifying, or such a case where the operating mode for the image process of the magnifying has been changed to that for the image process of the demagnifying.

As described above, the table 23 in the present embodiment is configured with a semiconductor device which can electrically rewrite the data on board, such as the EEPROM or the flash memory.

Moreover, table editing means for editing data of the parameters in the table 23 is formed with the CPU 22. The CPU 22 edits the parameters corresponding to an appropriate scope, which are paired with the scope ID, similarly to the case of reading the parameters.

Thereby, development of a new scope 2, improvement of an existing scope 2 and the like can be accommodated without modifying a board (hardware or software). Moreover, the development of the new scope 2, the improvement of the existing scope 2 and the like can be realized in a small-size circuit, and also excellent extensibility can be assured.

In order to more easily rewrite the data of the parameters and the like to be stored in the table 23 in this way, the configuration may be modified so that a personal computer (hereinafter abbreviated as "PC") 41 can be detachably connected to the processor 3 as shown in Fig. 7.

As shown in Fig. 7, in the processor 3, for example, a USB I/F 42 connected to the system bus 18 is further provided. The processor 3 is detachably connected to the PC 41 outside the processor 3 via a USB port of the USB I/F 42.

The PC 41 enables to rewrite the data in the table 23 via the USB port of the USB I/F 42 in the processor 3. It should be noted that other configuration in Fig. 7 is similar to that in Fig. 1 or Fig. 4.

Moreover, Fig. 8 shows an address allocation example in the table 23. Here, first, the parameters (data) of scope_1 to scope_m corresponding to m types of the scope 2 have been set. In this case, for example, the scope_1 has been set to have a paired relationship with a scope_1 ID (for example, the scope_1 ID includes a first address in which the parameters of the scope_1 have been stored, which is, of course, not limited to the example) as shown by a dotted line. Other scope_2 and the like have been set to have a similar relationship.

For example, the parameters of scope_3 corresponding to the scope 2 of a third type include common parameters, as well as individual preprocessing parameters, black balancing parameters, ..., and enhancement parameters (notation of the parameters is omitted in Fig. 8).

Then, for example, in order to accommodate a new scope, if the parameters of the scope (scope_new) are added, the parameters of the scope can be added, for example, in an area next to the parameters of the scope_m.

In that case, the CPU 22 records the scope ID corresponding to the new parameters (scope_new) and attributes of the area of the scope_new.

Moreover, in the case of the development of the scope, correction of the parameters or the like, the parameters related to an appropriate device of an appropriate scope are modified. As an example thereof, for example, Fig. 8 shows that the parameters of the AGC are rewritable.

As described above, the present embodiment has the registers for storing the parameters for the image processes for the scope types in the table 23 so that the parameters required for the processes can be written, and the functional modules for referring to the parameters written in the registers and performing calculation.

Thereby, in the common image processor 21 within the processor 3, it is possible to perform the suitable image processes depending on the type of the scope which is actually connected to the processor 3, without increasing its circuit configuration or a scope cable diameter.

Furthermore, since a function of observing (determining) a scope connection state is added, if the scope has been replaced corresponding to contents of an operation, an image processing state corresponding to the replaced scope can be quickly set at the processor 3 side. Therefore, according to the endoscope apparatus 1, if the scope has been replaced, it is possible to quickly set the endoscope apparatus 1 to an available state with the replaced scope.

In addition, since the table 23 for storing the parameters is a rewritable device, even if it is necessary to update the parameters of the existing scope or add the parameters to the new scope, the update or the addition of the parameters can be easily accommodated without requiring improvement or maintenance and repair such as the modification of the board or replacement of the ROM. Therefore, it is possible to reduce time and cost thereof. Moreover, an increase in a circuit size can be suppressed and also an endoscope apparatus having the excellent extensibility can be realized.

### (Second Embodiment)

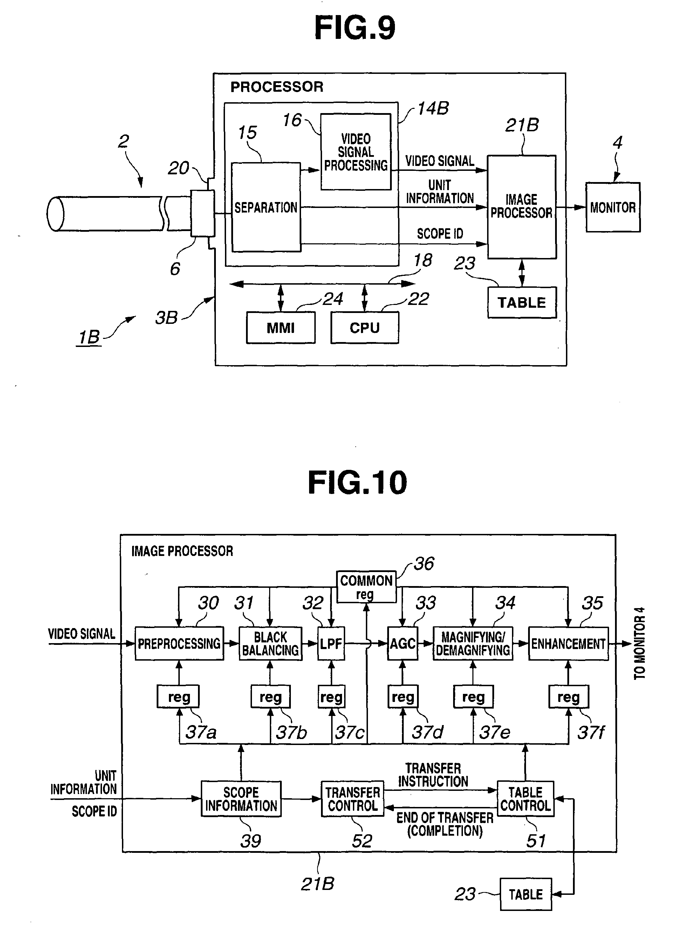

Next, with reference to Fig. 9, a second embodiment of the present invention will be described. Fig. 9 shows a configuration of an endoscope apparatus 1B including the second embodiment of the present invention. The present embodiment achieves the object similar to that of the first embodiment, and also achieves an object of reducing a load on the CPU 22 on startup and setting the endoscope apparatus to an operable state in a short time.

The endoscope apparatus 1B includes the scope 2, a processor 3B and the monitor 4. The processor 3B includes a scope I/F 14B including the separation block 15 and the video signal processing block 16, an image processor 21 B and the table 23, as well as the CPU 22 and the man-machine interface 24.

In this case, the CPU 22 is connected to the man-machine interface 24 via the system bus 18. The CPU 22 is configured so that the CPU 22 is not required to perform a control process such as reading the parameters based on the scope ID as described in the first embodiment. In other words, although the present embodiment includes a processing function similar to that of the first embodiment, the present embodiment has a configuration in which the CPU 22 is not required to perform the processing function (that is, a dedicated circuit performs the processing function).

The scope I/F 14B within the processor 3B has the separation block 15 similar to that of the first embodiment. The separation block 15 outputs the image pickup signal separated from the signal inputted from the scope 2, as the video signal to the image processor 21B via the video signal processing block 16. Moreover, the separation block 15 outputs the separated unit information and the separated scope ID to the image processor 21 B for performing the various image processes (video processes).

Moreover, the image processor 21 B is connected to the table 23 for storing the processing parameters required for the image processor 21B.

Fig. 10 shows an internal configuration of the image processor 21 B within the processor 3B of Fig. 9.

Similarly to the image processor 21 shown in Fig. 5, the image processor 2 1 B includes the preprocessing section 30, the black balancing section 31, the LPF section 32, the AGC section 33, the magnifying/demagnifying section 34 and the enhancement section 35, which perform the various image processes with respect to the video signal, and outputs the signal outputted from the enhancement section 35 to the monitor 4.

Moreover, similarly to the case of Fig. 5, the common register 36 in which the common parameters can be written and the respective individual registers 37a to 37f are included with respect to the preprocessing section 30, the black balancing section 31, the LPF section 32, the AGC section 33, the magnifying/demagnifying section 34 and the enhancement section 35.

The common register 36 and the registers 37a to 37f are connected to the scope information section 39 and a table control section 51.

In the first embodiment, depending on the scope ID from the scope 2 connected to the processor 3, the CPU 22 has controlled to read the corresponding parameters from the table 23 and transfer the corresponding parameters to the image processor 21.

In contrast to this, the present embodiment has a transfer control section 52 and the table control section 51 (which become dedicated circuits) within the image processor 21 B, and controls to read the corresponding parameters from the table 23 based on the scope ID and the unit information, write the corresponding parameters to the registers 37a to 37f within the image processor 21B and the like.

In the present embodiment, the unit information and the scope ID outputted from the scope I/F 14B are inputted to the scope information section 39 provided within the image processor 21B. The scope information section 39 is notified of the scope ID and the unit information from the scope I/F 14B when the power is turned on.

The scope information section 39 stores the unit information in an appropriate register and also notifies the transfer control section 52 provided within the image processor 21B that the scope information section 39 has received the scope ID.

The transfer control section 52 manages an order of which functional module the transfer is performed to. Specifically, the transfer control section 52 issues an instruction on a table area shown by the scope ID and a transfer destination module to the table control section 51.

The table control section 51 reads the parameter data corresponding to the register of the transfer destination module in the table area specified by the transfer control section 52, from the table 23, and transfers the parameter data to the register.

When the transfer to the register for the appropriate module is completed, the table control section 51 sends a completion notification to the transfer control section 52 and waits for the next instruction.

Similarly to the case of the first embodiment, the functional modules for performing calculation processes with respect to the video signal perform optimal processes depending on the scope 2 which is actually connected to the processor 3B, by performing the calculation processes with reference to the common register 36 and the registers 37a to 37f.

In the present embodiment, the scope information section 39 monitors a connected/not-connected state of the scope 2, for example, based on (whether or not there is any change in) the scope ID. For example, the scope information section 39 detects that the scope 2 has been replaced during operations, based on the change in the scope ID. If the scope ID has been changed, the scope information section 39 issues a transfer instruction with a new scope ID, with respect to the table control section 51.

Since the present embodiment has such a configuration, if the power is turned on, the present embodiment can reduce the load on the CPU 22 (than the first embodiment) and reduce the time for setting the endoscope apparatus to the available state.

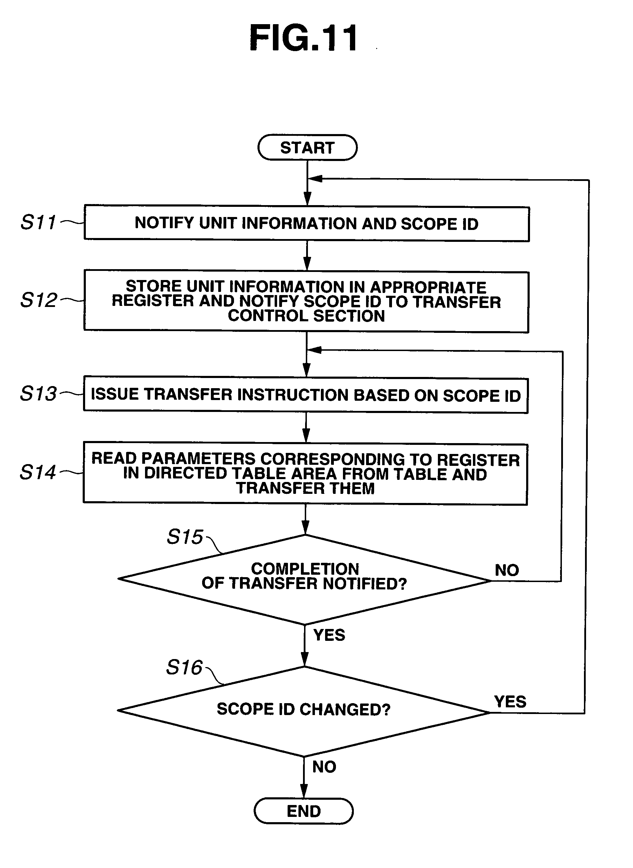

Fig. 11 shows a flowchart of contents of operations of the present embodiment. When the power is turned on and the processor 3B is activated, the unit information and the scope ID are notified to the scope information section 39 from the scope 2 side as shown in step S 11.

As shown in step S12, the scope information section 39 writes (sets) the unit information in the appropriate register (for example, 37a) and also notifies the scope ID to the transfer control section 52.

As shown in step S 13, the transfer control section 52 manages the order of which functional module the transfer is performed to, based on the notified scope ID, and issues the transfer instruction by issuing the instruction on the table area shown by the scope ID and the transfer destination functional module to the table control section 51.

As shown in step S 14, the table control section 51 reads the parameter data corresponding to the register of the transfer destination functional module in the table area directed by the transfer control section 52, from the table 23, transfers the parameter data to the register and writes the parameter data therein.

As shown in next step S 15, the transfer control section 52 waits until the completion of the transfer is notified by the table control section 51 to determine whether or not the transfer is completed. If the transfer is not completed, the processes from step S 13 to step S 15 are repeated.

When the completion of the transfer is notified, the process proceeds to next step S16. At step S 16, the scope information section 39 determines whether or not the scope ID has been changed, and if the scope ID has been changed during the operations, the operations from first step S 11 are performed. Thereby, even if the scope 2 has been replaced, it is possible to smoothly set an operating state for performing the suitable image processes, in a short time.

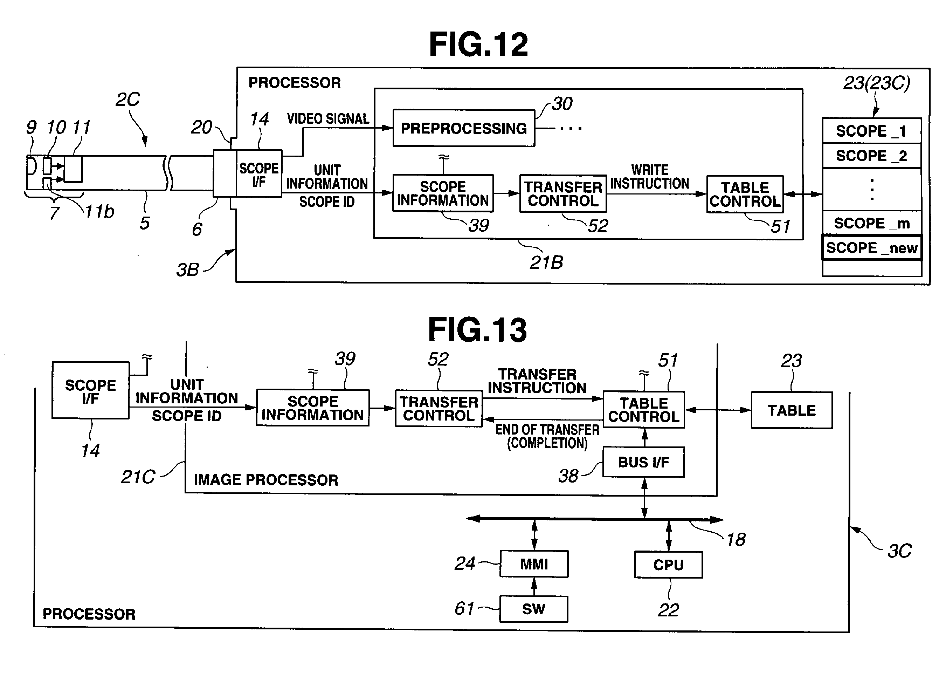

It should be noted that the table 23 in the present embodiment may be configured with an on-board rewritable device 23C such as a flash ROM and the like as shown in Fig. 12. Fig. 12 shows an example in which an unregistered (new) scope 2C has been connected. It should be noted that, in the example of Fig. 12, in the scope 2C, the ROM 1 b in which the parameters have been stored is provided, for example, separately from the multiplexing section 11, within the distal end portion 7 of the insertion portion 5. Moreover, the CPU 22 and the like are omitted in Fig. 12.

The transfer control section 52 determines whether the scope ID from the connected scope 2C is existing or new (unregistered), and if the transfer control section 52 determines that the scope 2C is the unregistered scope 2C, the transfer control section 52 additionally writes scope parameters stored in the ROM 11b within the scope 2C to the table 23 (via the instruction to the table control section 51), and registers the scope ID.

In other words, the operations in this case will be as follows.
(1) The scope information section 39 detects that the scope 2C or the like has been connected, based on its scope ID. The scope information section 39 notifies the scope ID to the transfer control section 52.
(2) The transfer control section 52 confirms the scope ID.
(3) The transfer control section 52 determines that the scope 2C is the unregistered scope 2C based on the scope ID, and receives the parameters from the scope 2C.
(4) The transfer control section 52 instructs the table control section 51 to write the parameters of the unregistered scope 2C. Then, the table control section 51 writes the parameters (scope_new in Fig. 12) in the rewritable device 23C.
(5) The transfer control section 52 registers the scope ID.

The present embodiment has similar advantages as those of the first embodiment.

Moreover, since the present embodiment has the table 23 in which the parameters for the image processes for the scope types have been stored, and has the configuration in which the parameters are transferred from the table 23 to the registers by the transfer control section 52 and the table control section 51 provided within the image processor 21B, the load on the CPU 22 at the time of turning on the power can be reduced. Also, time required for setting an operating state in which the user can perform the endoscopy can be reduced. Therefore, convenience for the user can be improved.

### (Third Embodiment)

Next, with reference to Fig. 13, a third embodiment of the present invention will be described. Fig. 13 shows a configuration of a periphery of the system bus 18 including a part of an image processor 21C. The present embodiment further achieves an object of enabling to quickly set an instructed operating state in response to an instruction from the user, for example, in the second embodiment and the like.

In the present embodiment, the configuration of the periphery of the system bus 18 which will be described below has been added in the configuration of Fig. 9 or Fig. 10.

A processor 3C according to the present embodiment has the image processor 21C in which the bus I/F 38 is further provided in the image processor 21B of Fig. 9, as shown in Fig. 13. The bus I/F 38 is connected to the system bus 18 to which the CPU 22 and the man-machine interface 24 are connected. Based on the instruction or the selection by the user, the image processes (based on the setting of the parameters) depending on the control from the CPU 22 side can be performed.

The man-machine interface 24 is an interface for a request (for example, to magnify or demagnify the displayed image, or to change its emphasis level or the like) made by operating a switch SW 61 or the like by the user during the operations, and notifies contents of the request to the CPU 22 via the system bus 18.

The CPU 22 notifies the contents of the request (a new magnifying power, a new emphasis level or the like) to the image processor 21C. The image processor 21C reads the parameters related to a new operating mode or processing mode notified by the CPU 22, for example, an enhancement coefficient in the case of changing the emphasis level, from the table 23, and sets the parameters in the register 37f for the enhancement functional module, and subsequently, starts the operations with the new emphasis level.

Then, the parameters of the related functional modules are set so that the process which is suitably corresponding to the request made by operating the switch SW 61 or the like can be performed.

Furthermore, the CPU 22 has means of, in response to the request to change the operating mode and the like from the user, notifying contents of the change to the transfer control section 52, and thereby time from the change request until start of the process with the changed mode is reduced, and an image corresponding to the requested process is displayed in a short time. In this way, it is possible to quickly accommodate the instruction from the user.

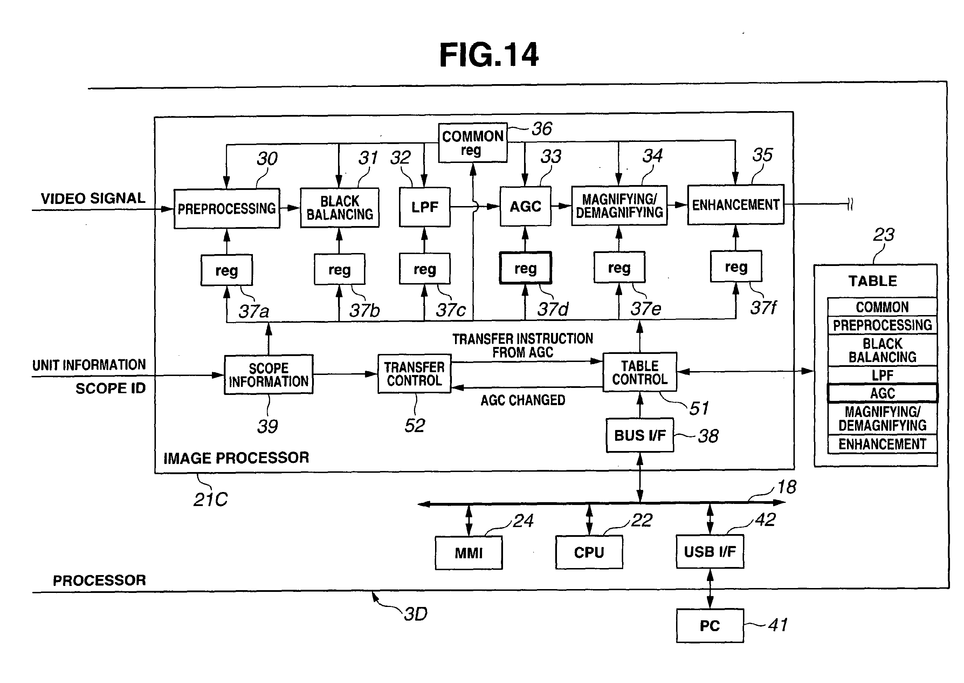

Fig. 14 shows a configuration of a processor 3D of a variation of Fig. 13. In the present variation, the processor 3D further has, for example, the USB I/F 42 in the configuration of Fig. 12, and the processor 3D is connected to the external PC 41 and the like so that the data in the table 23 can be rewritten from the PC 41.

If the data in the table 23 has been rewritten from the external PC 41, the transfer control section 52 determines which functional module rewritten parameters are related to, based on an accessed table address, and after the parameters are changed, the transfer instruction for the appropriate parameters is issued.

Fig. 14 shows a case where the parameters, for example, of the AGC section 33 in the image processing modules have been changed.

According to the present variation, when an evaluator changes the parameters, the change is reflected on image data processes by adding a function of determining which module the changed parameters are related to when the data is written in the table 23, based on its write address. Therefore, a procedure and a burden on the evaluator can be reduced. Moreover, the increase in the circuit size can be suppressed and also the excellent extensibility can be realized.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An endoscope apparatus to which an endoscope for transmitting a signal in which a video signal obtained by an image pickup section has been multiplexed with an endoscope ID as an endoscope identification signal is detachably connected, comprising:
an endoscope interface for separating the endoscope ID from the transmitted signal which has been received;
an image processor having a parameter register in which a parameter related to an image process of the video signal is set, and at least one functional module for performing a predetermined image process with respect to the video signal based on the parameter set in the parameter register;
a table memory for storing the parameter suitable for the endoscope corresponding to the endoscope ID for each endoscope ID; and
a parameter transfer control section for reading the parameter corresponding to the endoscope ID separated in the endoscope interface, from the table memory, and transferring the parameter to the parameter register.

2. The endoscope apparatus according to claim 1, wherein when the endoscope has been replaced, the parameter transfer control section obtains the endoscope ID of the replaced endoscope via the endoscope interface, reads the parameter corresponding to the obtained endoscope ID from the table memory, and transfers the parameter to the parameter register.

3. The endoscope apparatus according to claim 2, wherein the table memory is rewritable and, the endoscope apparatus further comprises a table memory editing section for editing the endoscope ID and the parameter related to the endoscope ID, which are paired in the table memory.

4. The endoscope apparatus according to claim 1, wherein the parameter transfer control section is provided as dedicated circuits for performing a parameter read function of reading the parameter from the table memory and a parameter transfer function of transferring the read parameter to the parameter register, respectively.

5. The endoscope apparatus according to claim 3, wherein the parameter paired with the endoscope ID is stored in the endoscope, and
the endoscope apparatus further comprises a determination section for determining whether or not the endoscope ID of the connected endoscope has been registered, and when the determination section determines that the endoscope ID has not been registered, the table memory editing section obtains the parameter from the connected endoscope via the endoscope interface and registers the endoscope ID and the obtained parameter as a pair in the table memory.

6. The endoscope apparatus according to claim 4, further comprising a mode setting section for setting an operating mode in the image processor.

7. The endoscope apparatus according to claim 6, wherein the parameter transfer control section reads the parameter corresponding to the set operating mode from the table memory and transfers the parameter to the parameter register.

8. The endoscope apparatus according to claim 1, wherein the table memory is rewritable and, the endoscope apparatus further comprises a table memory editing section for editing the parameter in the table memory, and
after the editing is performed by the table memory editing section, the parameter transfer control section reads the edited parameter from the table memory, and transfers the parameter to the parameter register related to the corresponding functional module.

9. The endoscope apparatus according to claim 1, wherein the endoscope interface, the image processor, the table memory and the parameter transfer control section are embedded in one processor to which the endoscope is detachably connected.

10. The endoscope apparatus according to claim 9, wherein the processor has a scope monitoring section for monitoring the connection of the endoscope to the processor and detaching of the endoscope connected to the processor.

11. The endoscope apparatus according to claim 10, wherein based on a result of the monitoring of the connection of the endoscope to the processor inputted by the scope monitoring section, the parameter transfer control section reads the parameter corresponding to the endoscope ID of the connected endoscope from the table memory, and transfers the parameter to the parameter register.

12. The endoscope apparatus according to claim 1, wherein the table memory is configured with a semiconductor memory which can electrically rewrite data to be stored.

13. The endoscope apparatus according to claim 1, wherein the image processor comprises at least two or more functional modules including a preprocessing section including color separation, a black level correction section for correcting a black level, a low pass filtering processing section for performing a low pass filtering process, an auto gain control processing section for performing automatic gain adjustment, a magnifying/demagnifying processing section for performing magnifying or demagnifying, and an enhancement processing section for performing an enhancement process, depending on parameters to be set respectively.

14. The endoscope apparatus according to claim 1, wherein the image processor is configured with a Digital Signal Processor for performing processes of two or more different functional modules.

15. The endoscope apparatus according to claim 1, wherein the parameter transfer control section and a parameter editing section for editing the parameter in the table memory are configured with a Central Processing Unit.

16. The endoscope apparatus according to claim 9, further comprising an external apparatus interface for editing the parameter in the table memory by an external apparatus outside the processor.

17. The endoscope apparatus according to claim 1, wherein the endoscope further multiplexes the transmitted signal which has been multiplexed with the endoscope ID, with device information on mounted devices including the image pickup section mounted on the endoscope.

18. The endoscope apparatus according to claim 17, wherein the endoscope interface separates the device information from the transmitted signal which has been multiplexed with the endoscope ID and the device information.

19. The endoscope apparatus according to claim 17, wherein the table memory further stores a parameter corresponding to the number of pixels and characteristics of the image pickup section corresponding to the device information.

20. The endoscope apparatus according to claim 19, wherein the parameter transfer control section further reads the parameter corresponding to the device information from the table memory and transfers the parameter to the parameter register.
